# EUROPÄISCHE PATENTSCHRIFT

(11) **EP 1 295 588 B1**
(45) Veröffentlichungstag und Bekanntmachung des Hinweises auf die Patenterteilung: **02.11.2005**
(21) Anmeldenummer: 02405530.3
(22) Anmeldetag: 25.06.2002
(51) Int. Cl.: A61K 7/00, A61K 7/06, A61K 7/48, A61K 7/50

(54) **Nachschäumendes kosmetisches Gel und Verfahren zu dessen Herstellung**
Post-foaming cosmetic gel and method for preparation thereof
Gel cosmétique moussant après application et procédé pour sa préparation

(30) Priorität: 25.09.2001 CH 176701
(43) Veröffentlichungstag der Anmeldung: 26.03.2003
(73) Patentinhaber: Mibelle AG Cosmetics, 5033 Buchs (CH)
(72) Erfinder: Karlen, Dr. Thomas, 5000 Aarau (CH); Irrgang, Bernhard, 5737 Menziken (CH)
(74) Vertreter: Rottmann, Maximilian

(56) Entgegenhaltungen:
- EP-A- 0 966 950
- WO-A-00/39273
- WO-A-97/03646
- WO-A-98/27936
- DE-A- 4 409 189
- US-A- 4 574 052
- US-A- 5 888 478

## Beschreibung

Die Erfindung betrifft ein nachschäumendes kosmetisches Gel gemäss Oberbegriff des Anspruchs 1.

Nachschäumende kosmetische Gele werden mit Hilfe einer geeigneten Verpackung gelförmig aus dem Aerosolbehälter auf die Haut aufgebracht und entwickeln erst dort nach kurzer Verzögerung unter dem Einfluss des Nachschäummittels den eigentlichen Schaum.

Zur Gelbildung bei der Vermischung der Tensidmasse mit dem Aufschäummittel werden üblicherweise polymere Verdickungsmittel oder tensidische Verdickungsmittel des nichtionischen Typs verwendet.

Die Verwendung von polymeren Verdickungsmitteln in Massen für nachschäumende seifenfreie Gele führt dazu, dass die Masse vor der Vermischung mit dem Aufschäummittel bereits eine hohe Viskosität aufweist. Dadurch ist die Durchmischung der tensidischen Masse mit dem Aufschäummittel erschwert. Zusätzlich können polymere Verdickungsmittel auf der Haut ein unangenehmes Klebegefühl vermitteln oder die Verteilbarkeit der Masse während der Anwendung negativ beeinflussen. Die Verwendung von polymeren Verdickungsmittel fördert überdies in keiner Weise die eigentlich gewünschte Klarheit des Gel-Produkts.

Die Verwendung von üblichen tensidischen Verdickungsmitteln wie beispielsweise Alkanolamiden, niedrig ethoxylierten Fettalkoholen oder -estern, ethoxylierten Fettsäuremono/di-glyceriden, Fettsäuremonoglyceriden führt bei der zur Gelbildung notwendigen Konzentration zu unerwünschten Anwendungseigenschaften, wie Fädenziehen oder Zähigkeit der Masse bei den rheologischen Eigenschaften, oder der Ausbildung einer trüben Masse bei den optischen Eigenschaften, oder zu einem starken Viskositätsaufbau der Masse vor Zugabe des Aufschäummittels, wodurch die Durchmischung mit dem Aufschäummittel stark erschwert wird.

Die WO 97/03646 A offenbart eine kosmetische nachschäumende Reinigungsgelzubereitung ein alkoxyliertes Amid, nämlich PEG-4 Rapeseedamide, in Kombination mit einem weiteren polymeren Verdickungsmittel enthält, und Isopentan als Nachschäummittel verwendet.

Die US Patentschrift US 5 888 478 setzt in transparenten, schäumenden kosmetischen Reinigungszubereitungen beispielsweise ethoxyliertes Stearinsäuremid als nicht-ionisches tensidisches Verdickunsmittel ein, und verwendet Isobutan als Nachschäummittel.

In der Offenlegungsschrift DE 44 09 189 hautmilde kosmetische Tensidzubereitungen mit guter Verdickbarkeit, gutem Schaumvermögen und Transparenz, die Kombinationen aus Alkylethercarboxylate mit alkokylierten Amiden, wie beispielweise dem ethoxylierten Rapsölfettsäureethanolamid, enthalten.

Die kosmetischen verzögert nachschäumenden Gelzubereitungen aus der US 5 334 325 enthalten als Nachschäummittel Mischungen aus kurzkettigen Kohlenwasserstoffen (bis 4 C-Atomen) und längerkettigen (5-8 C-Atome) Kohlenwasserstoffen.

Aufgabe der Erfindung war es daher, ein Mittel für die Reinigung der Haut oder der Haare in Form eines nachschäumenden Gels zu finden, welches die oben aufgeführten Nachteile nicht aufweist.

Diese Aufgabe wird durch die in den Kennzeichen der Ansprüche 1 und 7 umschriebenen Massnahmen gelöst. Bevorzugte Ausführungsformen sind in den übrigen Ansprüchen umschrieben.

Das erfindungsgemässe nachschäumende Gel, vorzugsweise als Duschgel oder Haarshampoo konfektioniert, enthält somit: (a) mindestens einen aliphatischen Kohlenwasserstoff mit einem Siedepunkt unter 50 °C als Nachschäummittel; (b) Wasser; gegebenenfalls (c) kosmetische Wirk- und Hilfsstoffe; und
**gekennzeichnet durch die Kombination folgender Merkmale,**
- es enthält als Nachschäummittel (a) ein Gemisch von Isopentan und Isobutan im Gewichtsverhältnis 2:1 bis 6:1;
- es enthält (d) 0,5 bis 9 Gew.-% mindestens eines alkoxylierten Amids als nichtionisches tensidisches Verdickungsmittel;
- das Gewichtsverhältnis von alkoxyliertem Amid (d) zum Nachschäummittel (a) beträgt 1:20 bis 2:1,
- es enthält höchstens 0,1 Gew.-% an polymeren Verdickungsmitteln;
- es ist frei von Seifen;
- es enthält (e) mindestens 0,5 Gew.-% eines neutralisierten Alkylethercarboxylates;

Das nichtionische tensidische Verdickungsmittel (d) besteht aus mindestens einem alkoxylierten Amid. Solche Amide weisen die Struktur I oder II auf.

(I): RC(O)-NH(CHR'CH₂O)ₙ-X n >= 2

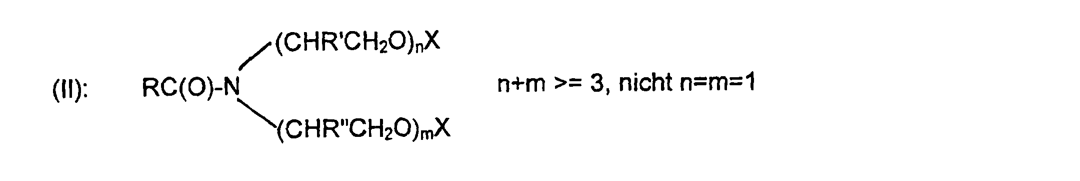

worin bedeuten:
- RC(O):: ein Fettsäureradikal
- R', R":: H oder einen Alkylrest
- X, X':: H oder ein beliebigen Substituenten wie z.B. eine Sulfosuccinylgruppe

Sein Gehalt beträgt zweckmässigerweise mindestens 0,1 Gew.-%, vorzugsweise 0,5 bis 9 Gew.-%.

Das Nachschäummittel (a) wird zweckmässigerweise in einer Menge von mindestens 0,5 Gew.-%, vorzugsweise von 1 bis 10 Gew.-% eingesetzt. Das Nachschäummittel trägt zum Aufschäumverhalten während der Anwendung bei.

Für eine optimale Gelbildung bei der Vermischung soll das Verhältnis der Komponente (d) zur Komponente (a) 1:20 und 2:1 betragen.

Bevorzugte alkoxylierte Amide (d) der Struktur I und II sind beispielsweise die folgenden nach der üblichen INCI-Deklaration bezeichneten Verbindungen:
- PEG-x Oleamide (x = 3 bis 20);
- PEG-x Cocamide (x = 2 bis 20),
- PEG-x Lauramide (x = 3 bis 20); und
- PEG-40 Ricinoleamide.

Besonders bevorzugt ist PEG-4 Rapeseedamide, welches unter dem Handelsnamen Aminol N™ von der Firma Kao vertrieben wird.

Die Ausführungsform des erfindungsgemässen nachschäumenden Gels enthält zusätzlich mindestens 0,5 Gew.-%, vorzugsweise 1 bis 15 Gew.-% eines Alkylethercarboxylates (e), welches mit einem geeigneten Neutralisationsmittel neutralisiert ist.

Beispiele für geeignete Alkylethercarboxylate sind die folgenden nach der üblichen INCI-Deklaration bezeichneten Verbindungen:
- Sodium Laureth-x Carboxylate (x = 11 bis 17),
- Sodium Trideceth-7 Carboxylate,
- Laureth-x Carboxylic Acid (x = 11 bis 17),
- Oleth-x Carboxylic Acid (x = 3 bis 6), als Trideceth-7 Carboxylic Acid bekannt, welches beispielsweise unter den Handelsbezeichnungen Akypo-R™-Typen der Firma Chem-Y, Akypo-Soft™-Typen der Firma Chem-Y, Rewopol-C™-Typen der Firma Rewo erhältlich ist.

Ein besonders bevorzugtes Alkylethercarboxylat ist beispielsweise das unter der Handelsbezeichnung Akypo-RLM-100™ (INCI: Laureth-11 Carboxylic Acid) der Firma Kao erhältliche Tensid. Durch die Mitverwendung eines Alkylethercarboxylates wird die Gelbildung beim Vermischen der tensidischen Masse mit dem Aufschäummittel weiter verbessert.

Geeignete Neutralisationsmittel zur Neutralisation des Alkylethercarboxylates (e) sind organische oder anorganische Basen. Als organische Basen sind beispielsweise primäre, sekundäre oder tertiäre Amine geeignet. Als anorganische Basen sind beispielsweise NaOH oder KOH geeignet.

Die erfindungsgemässen nachschäumenden Gele enthalten zweckmässigerweise 5 bis 30 Gew.-%, bezogen auf den Feststoffgehalt an Tensiden.

Bevorzugte Ausführungsformen der erfindungsgemässen nachschäumenden Gele enthalten neben dem Alkylethercarboxylat und dem tensidischen Verdickungsmittel (d) weitere nichtionische, anionische oder amphotere Tenside.

Bei der Herstellung der Gele geht man erfindungsgemäss so vor, dass man die Komponenten (b) und (d) sowie gegebenenfalls (c) und (e) zu einer tensidischen Grundmasse vermischt und diese anschliessend mit dem Nachschäummittel (b) unter Bildung eines Gels vermischt.

Die dynamische Viskosität der tensidischen Grundmasse (b + c + d + e) soll geeignet sein für eine rasche Vermischung mit dem Aufschäummittel (a). Dabei findet eine rheologische Änderung in Form einer Gelbildung statt. Die bei 20 °C bestimmte dynamische Viskosität der tensidischen Grundmasse (b + c + d + e) vor dem Vermischen mit dem Aufschäummittel (b) beträgt zweckmässigerweise weniger als 10'000 cP, vorzugsweise weniger als 3'000 cP.

Die bei 20 °C bestimmte dynamische Viskosität des nachschäumenden Gels nach der Vermischung der tensidischen Grundmasse (b + c + d + e) mit dem Nachschäummittel (a) beträgt zweckmässigerweise 1'000 bis 60'000 cP, vorzugsweise 2'000 bis 20'000 cP.

Der pH Bereich der erfindungsgemässen nachschäumenden Gele liegt zweckmässig zwischen 4,0 und 8,0.

Die erfindungsgemässen nachschäumenden Gele liegen im Moment der Entnahme aus einem geeigneten Dispenser als Paste, als Gel, vorzugsweise als klares Gel, vor, welches beim Kontakt mit der Haut bzw. der Kopfhaut einen kompakten und üppigen Schaum ausbildet.

Weiterhin können die erfindungsgemässen nachschäumenden Gele übliche kosmetische Zusatz- und Hilfsstoffe, z.B. Parfum, Konservierungsmittel oder Farbstoffe, sowie Wirkstoffe, insbesondere hautpflegende Substanzen, wie beispielsweise Proteine, enthalten. Diese Zusätze sind jedoch für die erfindungsgemässen nachschäumenden Gele nicht unbedingt erforderlich.

Insbesondere können sie beispielsweise folgende Zusatzstoffe enthalten:
- Öle;
- nichtionische, anionische, kationische oder amphotere Emulgatoren;
- Fette;
- Wachse;
- Siliconöle, insbesondere Cyclomethicone und Dimethicone Copolyole;
- Glycerin, Glykole, insbesondere Propylenglykol;
- organische oder anorganische Salze.

Die erfindungsgemässen nachschäumenden Gele *enthalten,* wie gesagt, höchstens 0,1 Gew.-% an Polymeren, deren primärer Einsatzzweck *das* Verdicken der Masse ist. Unter diesen Polymertyp fallen alle polymeren synthetischen oder natürlichen Verbindungen, welche in einer Konzentration von 0,1 Gew.-% in Wasser bei 20 °C eine dynamische Viskosität von mehr als 100 cP. Beispiele sind Celluloseether oder quervernetzte Acrylsäurepolymere.

Dies erfindungsgemässen nachschäumenden Gele können jedoch nichtionische, anionische oder amphotere nicht verdickende synthetische oder natürliche Polymere enthalten. Unter diesen Polymertyp fallen alle polymeren synthetischen oder natürlichen Verbindungen, welche in einer Konzentration von 0,1 Gew.-% in Wasser bei 20 °C eine dynamische Viskosität von weniger als 100 cP aufbauen.

Die Verbindungen sind nach der üblichen INCI-Deklaration bezeichnet. Alle Prozentangaben sind in Gew.-%.

### Beispiel 1

### Nachschäumendes Duschgel mit hautkonditionierender Wirkung

| | |
|---|---|
| Laurylethersulfat | 10,00 % |
| Laureth-11 Carboxylic Acid | 10,00 % |
| PEG-4 Rapeseedamide | 2,00 % |
| Isopentan | 6,00 % |
| Isobutan | 2,00 % |
| PEG-7 Glyceryl Cocoate | 1,00 % |
| Polyquaternium-7 | 0,10 % |
| Konservierung | q.s. |
| Wasser | ad 100 % |

Mit Natronlauge (30 %) auf pH 5 bis 7 einstellen.

### Beispiel 2

### Nachschäumendes Duschgel mit hautberuhigender Wirkung

| | |
|---|---|
| Laurylethersulfat | 12,00 % |
| Laureth-11 Carboxylic Acid | 8,00 % |
| Disodium Laureth Sulfosuccinate | 8.00 % |
| PEG-4 Rapeseedamide | 4,00 % |
| Isopentan | 6,00 % |
| Isobutan | 2,00 % |
| D-Panthenol | 1,00 % |
| Bisabolol | 0,10 % |
| Konservierung | q.s. |
| Wasser | ad 100 % |
| Mit Natronlauge (30 %) auf pH 5 bis 7 einstellen. | |

Mit Natronlauge (30 %) auf pH 5 bis 7 einstellen.
*Beispiel 3 fällt nicht unter die Ansprüche

### Beispiel 3*

### Nachschäumendes Gel für die Reinigung der Haare mit haarkonditionierender Wirkung

| | |
|---|---|
| Laurylethersulfat | 12,00 % |
| Laureth-11 Carboxylic Acid | 7,00 % |
| Cocamidopropyl Betaine | 4,00 % |
| PEG-4 Rapeseedamide | 3,00 % |
| Isopentan | 6,00 % |
| Isobutan | 2,00 % |
| Polyquaternium-10 | 0,30 % |
| Konservierung | q.s. |
| Wasser | ad 100 % |

Mit Natronlauge (30 %) auf pH 5 bis 7 einstellen.

### Beispiel 4

### Nachschäumendes Gel für die Reinigung der Haare mit kopfhautberuhigender Wirkung

| | |
|---|---|
| Laurylethersulfat | 12,00 % |
| Cocamidopropyl Betaine | 7,00 % |
| Laureth-11 Carboxylic Acid | 0,50 % |
| PEG-4 Rapeseedamide | 3,00 % |
| Isopentan | 6,00 % |
| Isobutan | 2,00 % |
| D-Panthenol | 1,00 % |
| Allantion | 0,20 % |
| Konservierung | q.s. |
| Wasser | ad 100 % |

Mit Triethanolamin auf pH 5 bis 7 einstellen.

### Beispiel 5

### Nachschäumendes Gel für die Reinigung der Haare mit Antischuppen-Wirkung

| | |
|---|---|
| Laurylethersulfat | 12,00 % |
| Laureth-11 Carboxylic Acid | 9,00 % |
| Cocamidopropyl Betaine | 3,00 % |
| PEG-4 Rapeseedamide | 2,00 % |
| Isopentan | 6,00 % |
| Isobutan | 2,00 % |
| Octopirox | 0,50 % |
| Allantion | 0,20 % |
| Konservierung | q.s. |
| Wasser | ad 100 % |

Mit Natronlauge (30 %) auf pH 5 bis 7 einstellen.

### Beispiel 6

### Nachschäumendes Duschgel mit Feuchtigkeitsspende für die Reinigung der empfindlichen Haut

| | |
|---|---|
| Laurylethersulfat | 10,00 % |
| Cocamidopropyl Betaine | 4,00 % |
| Laureth-11 Carboxylic Acid | 0,50 % |
| PEG-4 Rapeseedamide | 3,00 % |
| Isopentan | 6,00 % |
| Isobutan | 2,00 % |
| D-Panthenol | 1,00 % |
| Glycerin | 5,00 % |
| Allantion | 0,20 % |
| Konservierung | q.s. |
| Wasser | ad 100 % |

Mit Natronlauge (30 %) auf pH 5 bis 7 einstellen.

## Patentansprüche

1. Nachschäumendes kosmetische Gel, welches mindestens (a) einen aliphatischen Kohlenwasserstoff mit einem Siedepunkt unter 50 °C als Nachschäummittel; (b) Wasser; und gegebenenfalls (c) kosmetische Wirk- und Hilfsstoffe enthält, **gekennzeichnet durch die Kombination folgender Merkmale,**
- es enthält als Nachschäummittel (a) ein Gemisch von Isopentan und Isobutan im Gewichtsverhältnis 2:1 bis 6:1;
- es enthält (d) 0,5 bis 9 Gew.-% mindestens eines alkoxylierten Amids als nichtionisches tensidisches Verdickungsmittel;
- das Gewichtsverhältnis von alkoxyliertem Amid (d) zum Nachschäummittel (a) beträgt 1:20 bis 2:1,
- es enthält höchstens 0,1 Gew.-% an polymeren Verdickungsmitteln;
- es ist frei von Seifen;
- es enthält (e) mindestens 0,5 Gew.-% eines neutralisierten Alkylethercarboxylates;

2. Nachschäumendes kosmetisches Gel nach Anspruch 2, **dadurch gekennzeichnet, dass** es 1 bis 15 Gew.-% des neutralisierten Alkylethercarboxylates (e) enthält.

3. Nachschäumendes kosmetisches Gel nach einem der vorangehenden Ansprüche, **dadurch gekennzeichnet, dass** es bei 20 °C eine dynamische Viskosität von 1'000 bis 60'000 cP aufweist.

4. Nachschäumendes kosmetisches Gel nach Anspruch 6, **dadurch gekennzeichnet, dass** es bei 20 °C eine dynamische Viskosität von 2'000 bis 20'000 cP aufweist.

5. Nachschäumendes kosmetisches Gel nach einem der vorangehenden Ansprüche, **dadurch gekennzeichnet, dass** es ein pH von 4,0 bis 8,0 aufweist.

6. Duschgel nach einem der vorangehenden Ansprüche.

7. Haarshampoo nach einem der vorangehenden Ansprüche.

## Claims

1. Post-foaming cosmetic gel which comprises at least (a) an aliphatic hydrocarbon with a boiling point below 50°C as post-foaming agent; (b) water; and optionally (c) cosmetic active ingredients and auxiliaries, **characterized by the combination of the following features,**
- it comprises as post-foaming agent (a) a mixture of isopentane and isobutane in the weight ration 2:1 to 6:1;
- it comprises (d) 0.5 to 9% by weight of at least one alkoxylated amide as nonionic surface-active thickener;
- the weight ratio of alkoxylated amide (d) to the post-foaming agent (a) is 1:20 to 2:1,
- it comprises at most 0.1% by weight of polymeric thickeners;
- it is free from soaps;
- it comprises (e) at least 0.5% by weight of a neutralized alkyl ether carboxylate.

2. Post-foaming cosmetic gel according to Claim 2, **characterized in that** it comprises 1 to 15% by weight of the neutralized alkyl ether carboxylate (e).

3. Post-foaming cosmetic gel according to one of the preceding claims, **characterized in that** it has a dynamic viscosity at 20°C of from 1000 to 60 000 cP.

4. Post-foaming cosmetic gel according to Claim 6, **characterized in that** it has a dynamic viscosity at 20°C of from 2000 to 20 000 cP.

5. Post-foaming cosmetic gel according to one of the preceding claims, **characterized in that** it has a pH of from 4.0 to 8.0.

6. Shower gel according to one of the preceding claims.

7. Hair shampoo according to one of the preceding claims.

## Revendications

1. Gel cosmétique moussant après application, qui contient au moins (a) un hydrocarbure aliphatique avec un point d'ébullition inférieur à 50°C comme agent moussant après application ; (b) de l'eau, et le cas échéant, (c) des agents actifs et auxiliaires cosmétiques,
**caractérisé par** la combinaison des caractéristiques suivantes :
- il contient comme agent moussant après application (a), un mélange d'isopentane et d'isobutane en un rapport pondéral de 2:1 à 6:1 ;
- il contient (d) 0,5 à 9 % en poids d'au moins un amide alcoxylé comme agent épaississant tensioactif non ionique ;
- le rapport pondéral de l'amide alcoxylé (d) à l'agent moussant après application (a) se situe dans l'intervalle allant de 1:20 à 2:1 ;
- il contient au maximum, 0,1 % en poids d'agent épaississant polymère ;
- il est exempt de savon ;
- il contient (e) au moins 0,5 % en poids d'un carboxylate d'alkyléther neutralisé.

2. Gel cosmétique moussant après application selon la revendication 1, **caractérisé en ce qu'**il contient 1 à 15 % en poids du carboxylate d'alkyléther neutralisé (e).

3. Gel cosmétique moussant après application selon l'une des revendications précédentes, **caractérisé en ce qu'**il présente à 20°C, une viscosité dynamique de 1000 à 60 000 cP.

4. Gel cosmétique moussant après application selon la revendication 3, **caractérisé en ce qu'**il présente à 20°C, une viscosité dynamique de 2000 à 20 000 cP.

5. Gel cosmétique moussant après application selon l'une des revendications précédentes, **caractérisé en ce qu'**il présente un pH de 4,0 à 8,0.

6. Gel douche selon l'une des revendications précédentes.

7. Shampoing capillaire selon l'une des revendications précédentes.
